# EUROPEAN PATENT APPLICATION

(11) **EP 4 703 475 A1**
(43) Date of publication of application: **04.03.2026**
(21) Application number: 24796303.6
(22) Date of filing: 28.04.2024
(51) Int. Cl.: C12N 15/62, C12N 15/13, C12N 15/864, C12N 5/10, C12N 7/01, A61K 48/00, A61K 38/17, A61P 27/02, C12R 1/93

(54) **NUCLEIC ACID ENCODING ANTI-VEGF PROTEIN, POLYNUCLEOTIDE EXPRESSION CASSETTE AND RECOMBINANT ADENO-ASSOCIATED VIRUS**

(30) Priority: 28.04.2023 CN 202310483755; 26.07.2023 CN 202310930549
(71) Applicant: Chengdu Origen Biotechnology Co., Ltd., Chengdu, Sichuan 610037 (CN)
(72) Inventor: KE, Xiao, Chengdu, Sichuan 610036 (CN); LUO, Shuang, Chengdu, Sichuan 610036 (CN); ZHENG, Qiang, Chengdu, Sichuan 610036 (CN)
(74) Representative: Michalski Hüttermann & Partner Patentanwälte mbB
(86) International application number: PCT/CN2024/090274
(87) International publication number: WO 2024/222934

(57) **Abstract**

Provided are a nucleic acid encoding an anti-VEGF protein, a polynucleotide expression cassette, a recombinant adeno-associated virus, and the related use thereof. By means of further optimizing and designing an AAV vector for encoding an anti-VEGF protein, the expression level of the protein is improved, and the potential clinical value thereof is better exerted.

## Description

### TECHNICAL FIELD

The present disclosure relates to the fields of genetic engineering/biopharmaceuticals, and particularly, the present disclosure relates to a nucleic acid encoding an anti-VEGF protein, a polynucleotide expression cassette, a recombinant adeno-associated virus, and related use thereof.

### BACKGROUND

Neovascularization is a key factor in the development and spread of many diseases. Many ocular diseases involve angiogenesis, including age-related macular degeneration (AMD), retinal vein occlusion (RVO), diabetic retinopathy (DR), pathological myopia, etc. VEGF is a highly specific promoter of vascular endothelial cell growth, which functions to promote increased vascular permeability, extracellular matrix degeneration, vascular endothelial cell migration and proliferation, and blood vessel formation. Numerous studies have confirmed that excessive VEGF expression can induce pathological neovascular ocular diseases. Given the importance of VEGF signaling in angiogenesis, blocking VEGF or VEGF receptors to inhibit angiogenesis has a significant therapeutic effect on diseases related to angiogenesis, including cancer, retinal vascular lesion, etc. Over the past decade, some anti-VEGF drugs have been developed for the treatment of ocular neovascular diseases, such as pegaptanib (Macugen), bevacizumab (Avastin), ranibizumab (Lucentis), and aflibercept (Eylea).

However, due to the short half-life of currently available drugs, repeated administrations are required, which increases the burden on both doctors and patients. Therefore, gene therapy approaches, owing to their ability to maintain long-term efficacy through sustained drug expression, have been regarded with great expectations, such as adeno-associated virus (AAV) vector-based gene therapy tools. However, there are still some challenges in the application of AAV, such as injection site irritation and immune responses caused by dosage issues. Thus, further optimization of AAV vectors is desired.

### CONTENT OF THE PRESENT INVENTION

In order to solve the problems described above, the present disclosure conducts extensive optimization on a coding sequence expressing an anti-VEGF protein, such as codon optimization, CpG removal, and intron insertion in the coding sequence region, with the aim of enhancing the expression level of the anti-VEGF protein. For example, in some preferred embodiments, the coding sequence has been subjected to codon optimization. The present disclosure provides a coding sequence that has been subjected to codon optimization, wherein the coding sequence is subjected to codon modification by methods well known in the art on the basis of not changing the amino acid sequence of the encoded product, for example, by replacing codons with low-frequency expression with codons with high-frequency expression in target cells. In some preferred embodiments, the coding sequence is a coding sequence that has been subjected to CpG removal. Changing the composition of the CpG island or the GC content of the coding region may influence the selection of codon usage preference. In other preferred embodiments, the coding sequence is a coding sequence that has been subjected to CpG removal and codon optimization. In some preferred embodiments, the coding sequence encoding an anti-VEGF protein described in the present disclosure comprises codon optimization and/or CpG removal and/or intron insertion.

One aspect of the present disclosure is to provide a nucleic acid, wherein the nucleic acid comprises a coding sequence encoding an anti-VEGF protein, and the coding sequence has been subjected to codon optimization.

In some embodiments, the anti-VEGF protein described in the present disclosure comprises extracellular domain 2 of VEGF receptor 1 and extracellular domain 3 of VEGF receptor 2. In certain embodiments, the anti-VEGF protein described in the present disclosure further comprises extracellular domain 4 of VEGF receptor 2. In certain embodiments, the anti-VEGF protein described in the present disclosure further comprises an immunoglobulin Fc fragment (e.g., human immunoglobulin IgG1, IgG2, or IgG4).

In some embodiments, the anti-VEGF protein described in the present disclosure comprises extracellular domain 2 of VEGF receptor 1, extracellular domain 3 of VEGF receptor 2, and an immunoglobulin Fc fragment. In other embodiments, the anti-VEGF protein of the present disclosure comprises extracellular domain 2 of VEGF receptor 1, extracellular domain 3 of VEGF receptor 2, extracellular domain 4 of VEGF receptor 2, and an immunoglobulin Fc fragment.

In some specific embodiments, the anti-VEGF protein described in the present disclosure comprises SEQ ID NO: 1, SEQ ID NO: 2, or a protein having at least 85% (e.g., 85%, 90%, 95%, 98%, 99%, or 100%) homology to either of the sequences.

In some specific embodiments, the nucleic acid that has been subjected to codon optimization comprises the nucleotide sequence set forth in SEQ ID NO: 7 or a nucleotide sequence having at least 85% (e.g., 85%, 90%, 95%, 98%, 99%, or 100%) homology thereto.

In some preferred embodiments, the nucleic acid molecule of the coding sequence has been further subjected to CpG removal. In some embodiments, at least 1, 2, 3, 4, 5, 6, 7, 8, 9, 10, 20, 30, 40, 50 CpGs may be removed compared to a coding sequence without CpG removal. In some embodiments, at least 10%, 20%, 30%, 40%, 50%, 60%, 70%, 80%, 90%, or 100% of CpG may be removed compared to a coding sequence without CpG removal. In some preferred specific embodiments, the nucleic acid of the coding sequence comprises the nucleotide sequence set forth in SEQ ID NO: 8 or a nucleic acid sequence having at least 85% (e.g., 85%, 90%, 95%, 98%, 99%, or 100%) homology thereto.

In some embodiments, the nucleic acid molecule of the coding sequence described in the present disclosure further comprises an intron insertion. In some specific embodiments, the intron is selected from: a VH4 intron, an SV40 intron, a Chi intron, and a Chimeric intron. In some specific embodiments, the VH4 intron, the SV40 intron, the Chi intron, and the Chimeric intron have the nucleotide sequence set forth in SEQ ID NO: 3, SEQ ID NO: 4, SEQ ID NO: 5, or SEQ ID NO: 20, respectively.

In some specific embodiments, the coding sequence encoding the anti-VEGF protein has been subjected to codon optimization, CpG removal, and intron insertion simultaneously. In some preferred embodiments, the coding sequence encoding the anti-VEGF protein comprises the nucleotide sequence set forth in SEQ ID NO: 6, SEQ ID NO: 13, SEQ ID NO: 21, or SEQ ID NO: 22 or a nucleic acid sequence having at least 85% homology thereto.

Another aspect of the present disclosure is to provide a polynucleotide expression cassette, which comprises the nucleic acid described in the present disclosure and an expression regulatory element (wherein as described above, the nucleic acid described in the present disclosure comprises a coding sequence encoding an anti-VEGF protein).

In a specific embodiment, the polynucleotide expression cassette described in the present disclosure comprises, in a 5' to 3' order:
1) a 5' ITR;
2) a promoter;
3) an intron;
4) the aforementioned nucleic acid encoding the anti-VEGF protein of the present disclosure;
5) a polyadenylation signal sequence;
6)a3'ITR.

The promoter is selected from a β-actin (CBA) promoter, a cytomegalovirus (CMV) promoter, an elongation factor 1α (EF1α) promoter, an EFS promoter, an NA65P promoter, an MNT promoter, a UB6 promoter, a CAG promoter, an RPE65 promoter, a UBC promoter, or an opsin promoter, preferably a CBA, CMV, EF1α, or CAG promoter.

The intron is selected from a VH4 intron, an SV40 intron, a Chi intron, a chicken β-actin intron, a U12 intron, an RHD intron, a PI intron, an MBL intron, and the like, preferably a VH4 intron, an SV40 intron, a Chi intron, and a chicken β-actin intron.

The polyadenylation signal includes simian vacuolating virus 40 (SV40), human growth hormone (HGH), bovine growth hormone (BGH), or β-globin (RGB).

In some specific embodiments, the nucleic acid encoding the anti-VEGF protein encodes SEQ ID NO: 1 or SEQ ID NO: 2 or an amino acid sequence having at least 85% homology thereto.

In some more specific embodiments, the nucleic acid encoding the anti-VEGF protein comprises SEQ ID NO: 6, SEQ ID NO: 7, SEQ ID NO: 8, SEQ ID NO: 13, SEQ ID NO: 21, SEQ ID NO: 22, or a nucleotide sequence having at least 85% homology thereto.

In some more specific embodiments, the polynucleotide expression cassette described in the present disclosure comprises, in a 5' to 3' order:
1) a 5' ITR;
2) a CBA promoter;
3) a chicken β-actin intron;
4) a kozak sequence;
5) a nucleic acid encoding an anti-VEGF protein, wherein the nucleic acid encodes SEQ ID NO: 1 or SEQ ID NO: 2 or an amino acid sequence having at least 85% homology thereto;
6) an RGB polyadenylation signal sequence;
7)a3'ITR.

In some more specific embodiments, the polynucleotide expression cassette described in the present disclosure comprises, in a 5' to 3' order:
1) a 5' ITR;
2) a CBA promoter;
3) a chicken β-actin intron;
4) a kozak sequence;
5) a nucleic acid encoding an anti-VEGF protein, wherein the nucleic acid comprises SEQ ID NO: 6, SEQ ID NO: 7, SEQ ID NO: 8, SEQ ID NO: 13, SEQ ID NO: 21, SEQ ID NO: 22, or a nucleotide sequence having at least 85% homology thereto;
6) an RGB polyadenylation signal sequence;
7)a3'ITR.

In some specific embodiments, the nucleic acid encoding the anti-VEGF protein in the polynucleotide expression cassette has been subjected to codon optimization. In some more specific embodiments, the polynucleotide expression cassette (the nucleic acid encoding the anti-VEGF protein has been subjected to codon optimization) comprises, from 5' to 3', the nucleotide sequence set forth in SEQ ID NO: 27 or a sequence having at least 85% (e.g., 85%, 90%, 95%, 98%, 99%, or 100%) homology thereto.

In some specific embodiments, the nucleic acid encoding the anti-VEGF protein in the polynucleotide expression cassette has been subjected to CpG removal. In some more specific embodiments, the polynucleotide expression cassette (the nucleic acid encoding the anti-VEGF protein has been subjected to codon optimization and CpG removal) comprises, from 5' to 3', the nucleotide sequence set forth in SEQ ID NO: 29 or a sequence having at least 85% (e.g., 85%, 90%, 95%, 98%, 99%, or 100%) homology thereto.

In some specific embodiments, the nucleic acid encoding the anti-VEGF protein in the polynucleotide expression cassette further comprises an intron insertion. The intron illustratively includes, but is not limited to, a VH4 intron, an SV40 intron, a Chi intron, and a Chimeric intron. In some preferred embodiments, the nucleic acid encoding the anti-VEGF protein comprises an insertion of a VH4 intron, and the polynucleotide expression cassette comprises, from 5' to 3', the nucleotide sequence set forth in SEQ ID NO: 9 or SEQ ID NO: 31 or a nucleic acid sequence having at least 85% homology thereto. In some preferred embodiments, the nucleic acid encoding the anti-VEGF protein comprises an insertion of an SV40 intron, and the polynucleotide expression cassette comprises, from 5' to 3', the nucleotide sequence set forth in SEQ ID NO: 23 or a nucleic acid sequence having at least 85% homology thereto. In some preferred embodiments, the nucleic acid encoding the anti-VEGF protein comprises an insertion of a Chimeric intron, and the polynucleotide expression cassette comprises, from 5' to 3', the nucleotide sequence set forth in SEQ ID NO: 24 or a nucleic acid sequence having at least 85% homology thereto.

In some more specific embodiments, the polynucleotide expression cassette described in the present disclosure comprises, in a 5' to 3' order:
1) a 5' ITR;
2) a CBA promoter;
3) a chicken β-actin intron;
4) a kozak sequence;
5) a nucleic acid encoding an anti-VEGF protein, wherein the nucleic acid encodes SEQ ID NO: 1 or SEQ ID NO: 2 or an amino acid sequence having at least 85% homology thereto;
6) an SV40 polyadenylation signal sequence;
7)a3'ITR.

In some more specific embodiments, the polynucleotide expression cassette described in the present disclosure comprises, in a 5' to 3' order:
1) a 5' ITR;
2) a CBA promoter;
3) a chicken β-actin intron;
4) a kozak sequence;
5) a nucleic acid encoding an anti-VEGF protein, wherein the nucleic acid comprises SEQ ID NO: 6 or a nucleotide sequence having at least 85% homology thereto;
6) an SV40 polyadenylation signal sequence;
7)a3'ITR.

In some more specific embodiments, the polynucleotide expression cassette comprises, from 5' to 3', the nucleotide sequence set forth in SEQ ID NO: 33 or a sequence having at least 85% (e.g., 85%, 90%, 95%, 98%, 99%, or 100%) homology thereto.

Another aspect of the present disclosure is to provide a plasmid, wherein the plasmid comprises the aforementioned nucleic acid encoding the anti-VEGF protein or polynucleotide expression cassette of the present disclosure.

In some preferred embodiments, the plasmid described in the present disclosure has the nucleotide sequence set forth in SEQ ID NO: 10, SEQ ID NO: 28, SEQ ID NO: 30, SEQ ID NO: 32, or SEQ ID NO: 34 or a sequence having at least 85% homology thereto.

Another aspect of the present disclosure is to provide a recombinant adeno-associated virus (rAAV), wherein the recombinant adeno-associated virus comprises a capsid protein and the aforementioned polynucleotide expression cassette of the present disclosure.

In some preferred embodiments, the capsid protein is a wild-type or modified capsid protein selected from any one of the capsid proteins of AAV1, AAV2, AAV3, AAV4, AAV5, AAV6, AAV7, AAV8, AAV9, AAV10, AAV11, and AAV12. In some more preferred embodiments, the capsid protein is an AAV8 capsid protein or a modified AAV8 capsid protein. In some preferred embodiments, the modified capsid protein comprises a heterologous polypeptide substitution of about 5-14 amino acids at amino acid positions 588-592 of the parental AAV8 (the amino acids at positions 588-592 of the parental/wild-type AAV8 capsid protein are "QQNTA"), such as a polypeptide sequence RGNQQNTARQ (SEQ ID NO: 11).

Another aspect of the present disclosure is to provide a host cell, wherein the host cell comprises the aforementioned nucleic acid encoding the anti-VEGF protein or polynucleotide expression cassette or plasmid or recombinant adeno-associated virus of the present disclosure.

Another aspect of the present disclosure is to provide a pharmaceutical composition. The pharmaceutical composition comprises the polynucleotide expression cassette, the plasmid, or the recombinant virus of the present disclosure, and a pharmaceutically acceptable excipient. In some embodiments, the pharmaceutical composition comprises the recombinant virus described in the present disclosure and a pharmaceutically acceptable excipient. In some embodiments, the pharmaceutical composition is prepared as a formulation for intravitreal injection, subretinal injection, suprachoroidal space injection, intravenous injection, intratumoral injection, or intramuscular injection.

Another aspect of the present disclosure is to provide use of the polynucleotide expression cassette, the plasmid, or the recombinant virus described in the present disclosure in the manufacture of a medicament for treating a VEGF-associated disease. In certain embodiments, the VEGF-associated disease is an ocular neovascular disease. In certain embodiments, the ocular neovascular disease is selected from age-related macular degeneration, diabetic retinopathy, diabetic macular edema, central retinal vein occlusion, macular edema caused by branch retinal vein occlusion, macular edema secondary to retinal vein occlusion, polypoidal choroidal vasculopathy, wet age-related macular degeneration with very low vision, and choroidal neovascularization secondary to pathological myopia.

Another aspect of the present disclosure is to provide a method for treating a VEGF-associated disease in a mammalian subject, wherein the method comprises delivering a therapeutically effective amount of the polynucleotide expression cassette, the plasmid, or the recombinant virus described in the present disclosure to the eye. In certain embodiments, the VEGF-associated disease is an ocular neovascular disease. In certain embodiments, the ocular neovascular disease is selected from age-related macular degeneration, diabetic retinopathy, diabetic macular edema, central retinal vein occlusion, macular edema caused by branch retinal vein occlusion, macular edema secondary to retinal vein occlusion, polypoidal choroidal vasculopathy, wet age-related macular degeneration with very low vision, and choroidal neovascularization secondary to pathological myopia.

In some embodiments, the recombinant virus or the pharmaceutical composition described in the present disclosure is administered via intravitreal injection, subretinal injection, suprachoroidal space injection, intravenous injection, intratumoral injection, or intramuscular injection.

In some embodiments, the recombinant adeno-associated virus is administered at a dose of E8/eye to E16/eye. In some preferred embodiments, the recombinant adeno-associated virus is administered at a dose of 5E11/eye to 2E12/eye. In some more preferred embodiments, the recombinant adeno-associated virus is administered at a dose of 2E12/eye.

### DETAILED DESCRIPTION

In the present disclosure, the following terms have the following definitions.

As described herein, "polynucleotide expression cassette" refers to two or more functional nucleotide sequences that are operably linked to each other. The nucleotide sequences, e.g., expression regulatory elements, translation initiation sequence, coding sequences, termination sequences, etc., are generally composed of DNA.

As described herein, "coding sequence" refers to a nucleotide sequence that encodes a gene product *in vivo* or *in vitro.* A coding sequence or gene may encode a polypeptide or a protein molecule.

As described herein, "promoter" refers to a DNA sequence that directs the binding of RNA polymerase and thereby promotes RNA synthesis. A promoter and its corresponding protein expression may be universal (meaning strongly active in cells, tissues, and species) or exhibit cell-specificity, tissue-specificity, or species-specificity.

As described herein, "intron" may refer to any sequence that can be transcribed but not translated. In some cases, an intron may refer to any sequence that is transcribed and removed from a mature RNA transcript in a cell.

The term "operably linked" refers to the juxtaposition of genetic elements in a relationship that allows them to operate in an intended manner. For example, a promoter is operably linked to a coding region if it helps initiate transcription of the coding sequence. As long as this function is maintained, there may be intermediate residues between the promoter and the coding region.

The term "inverted terminal repeat (ITR)" refers to an ITR sequence used to replicate and package an AAV virus.

The term "AAV" is an abbreviation for adeno-associated virus and is used to refer to the virus itself or a derivative thereof. The term includes all viral subtypes as well as viruses that exist in their native and recombinant forms. The AAV includes AAV type 1 (AAV-1), AAV type 2 (AAV-2), AAV type 3 (AAV-3), AAV type 4 (AAV-4), AAV type 5 (AAV-5), AAV type 6 (AAV-6), AAV type 7 (AAV-7), AAV type 8 (AAV-8), AAV type 9 (AAV-9), type 10 (AAV-10), type 11 (AAV-11), type 12 (AAV-12), avian AAV, bovine AAV, canine AAV, equine AAV, primate AAV, non-primate AAV, and ovine AAV. "Primate AAV" refers to an AAV that infects primates, "non-primate AAV" refers to an AAV that infects non-primates, etc.

The term "variant" refers to a mutant of a reference nucleotide or amino acid, which, for example, has at least one nucleotide or amino acid difference (e.g., an insertion, substitution, or deletion of an amino acid or nucleotide) relative to the sequence of a natural nucleotide or amino acid. The nucleotide or amino acid mutant described in the present disclosure may also be described as a modified amino acid or nucleotide.

The term "comprise" or "comprising" means that in addition to the necessary elements in the subject matter, elements not mentioned in the subject matter can also be encompassed. For example, a polynucleotide expression cassette comprising a promoter may also comprise other elements (e.g., an ITR, an enhancer, an intron, a coding gene, a polyadenylation sequence, etc.) in addition to the promoter.

The terms "polyadenylation sequence", "polyadenylation region", and "polyadenylation signal" comprise the recognition region required for an endonuclease to cleave an RNA transcript, followed by the polyadenylation consensus sequence AATAAA. The polyadenylation sequence provides a PolyA site, which is the site on the RNA transcript where adenine residues are added through post-transcriptional polyadenylation.

The term "Kozak sequence" refers to a segment of nucleic acid sequence located downstream of a 5' cap structure in eukaryotic mRNA, generally GCCACCAUGG, and it can bind to a translation initiation factor to mediate the initiation of translation for mRNA containing a 5' cap structure. It functions in the initiation of protein translation.

The term "homology" refers to the degree to which amino acid sequences or base sequences are similar when two or more nucleotide sequences or amino acid sequences are aligned, expressed as a percentage, such as 85%, 90%, 95%, 99%, or 100%.

The term "host cell" refers to a cell transduced, infected, transfected, or transformed with a vector. The vector may be a plasmid, a viral particle, a phage, or the like. It should be understood that the term "host cell" refers to the original transduced, infected, transfected, or transformed cell and progeny thereof.

The term "transduced", "infected", "transfected", or "transformed" generally refers to a method for administering, introducing, or inserting exogenous DNA (vector) into a cell. When DNA is introduced into a cell by a virus or viral vector, the cell is transduced with exogenous DNA. When DNA is introduced into a cell by non-viral methods, the cell is transfected with exogenous DNA. The terms "transduced" and "infected" are used interchangeably herein to refer to cells that have received exogenous DNA or polynucleotides from a virus or viral vector.

The term "VEGF-associated disease" refers to a related disease caused by abnormal expression (e.g., excess or deficiency) of VEGF.

The term "expression regulatory element" refers to a segment of nucleic acid sequence that regulates the expression of a nucleotide sequence operably linked thereto. When a segment of expression regulatory element controls and regulates the transcription and/or translation of a segment of nucleotide sequence, the expression regulatory element is "operably linked" to the nucleotide sequence. Therefore, expression regulatory elements may comprise a promoter, an enhancer, an internal ribosome entry site (IRES), a transcription terminator, an initiation codon preceding protein-coding genes, an intron splicing signal, and a termination codon. The term "expression regulatory element" is intended to comprise, at a minimum, a segment of sequence present for the purpose of influencing expression, and may also comprise other advantageous components. The term may also comprise a nucleic acid sequence design that removes undesired potential initiation codons, whether in-frame or out-of-frame, from the sequence. It may also comprise a nucleic acid sequence design that removes undesired potential splice sites. It may also comprise a sequence that directs the addition of polyadenylation or a polyA.

The term "codon optimization" refers to the modification of a nucleic acid sequence encoding the same product by using a synonymous codon sequence, for example, by replacing codons with low-frequency expression with codons with high-frequency expression in target cells or species, with the aim of enhancing the expression level of the target gene in the target cells, tissues, or species (e.g., by 10%, 20%, 30%, 40%, 50%, 60%, 70%, 80%, 90%, 100%, or more than 100%). Any nucleic acid modification that contributes to increased expression of the same encoded product by the target gene in the target cells or species falls within the scope of codon optimization according to the present disclosure.

### Vector

In order to increase the level of the target gene encoding or expressing the therapeutic protein or antibody, the coding sequence expressing the anti-VEGF protein is extensively optimized in the present disclosure via, for example, codon optimization, CpG removal, intron insertion in the coding sequence region, and the like to allow an expression vector (e.g., a nucleic acid, a polynucleotide expression cassette, a plasmid, or a recombinant adeno-associated virus) to have increased infectivity or expression ability for target tissues or target cells (e.g., the retina). For example, in some embodiments, the expression level of the anti-VEGF protein of the expression vector in the target cells or target tissues is increased by 10%, 20%, 30%, 40%, 50%, 60%, 70%, 80%, 90%, 100%, 150%, 200%, 300%, 400%, 500%, or more relative to that of an non-optimized vector.

Therefore, one aspect of the present disclosure is to provide a nucleic acid vector, wherein the nucleic acid comprises a coding sequence encoding an anti-VEGF protein, and the coding sequence has been subjected to codon optimization. The present disclosure provides a coding sequence that has been subjected to codon optimization, wherein the coding sequence is subjected to codon modification by methods well known in the art on the basis of not changing the amino acid sequence of the encoded product, for example, by replacing codons with low-frequency expression with codons with high-frequency expression in target cells.

The anti-VEGF protein of the present disclosure targets (e.g., specifically binds to) a human VEGF receptor. VEGF receptors are receptors for vascular endothelial growth factors (VEGFs). There are three major subtypes of VEGF receptors, namely VEGFR-1, VEGFR-2, and VEGFR3. VEGF receptors have an extracellular portion composed of 7 immunoglobulin-like domains (e.g., extracellular domains 1-7), a single transmembrane spanning region, and an intracellular portion containing a split tyrosine kinase domain.

In some embodiments, the anti-VEGF protein of the present disclosure is a human VEGF receptor or a portion of a receptor. It is capable of recognizing and binding to a VEGF ligand but is not capable of activating a receptor complex, thus exerting an inhibitory effect, binding to the ligand, and preventing the ligand from binding to a conventional receptor. For example, the anti-VEGF protein of the present disclosure is any one of extracellular domains 1-7 of human VEGF receptor 1, or a combination of multiple domains. For another example, the anti-VEGF protein of the present disclosure is any one of extracellular domains 1-7 of human VEGF receptor 2, or a combination of multiple domains. For another example, the anti-VEGF protein of the present disclosure is any one of extracellular domains 1-7 of human VEGF receptor 3, or a combination of multiple domains. For another example, the anti-VEGF protein of the present disclosure is a combination of one or more extracellular domains of domains 1-7 of human VEGF receptor 1, and/or VEGF receptor 2, and/or VEGF receptor 3.

In some embodiments, the anti-VEGF protein described in the present disclosure comprises extracellular domain 2 of VEGF receptor 1 and extracellular domain 3 of VEGF receptor 2. In certain embodiments, the anti-VEGF protein described in the present disclosure further comprises extracellular domain 4 of VEGF receptor 2. In certain embodiments, the anti-VEGF protein described in the present disclosure further comprises an immunoglobulin Fc fragment (e.g., human immunoglobulin IgG1, IgG2, or IgG4).

In some embodiments, the anti-VEGF protein described in the present disclosure comprises extracellular domain 2 of VEGF receptor 1, extracellular domain 3 of VEGF receptor 2, and an immunoglobulin Fc fragment. In other embodiments, the anti-VEGF protein of the present disclosure comprises extracellular domain 2 of VEGF receptor 1, extracellular domain 3 of VEGF receptor 2, extracellular domain 4 of VEGF receptor 2, and an immunoglobulin Fc fragment.

The protein described in the present disclosure includes a polypeptide, a fusion protein, an antibody, or a functional fragment thereof.

In some specific embodiments, the anti-VEGF protein described in the present disclosure comprises SEQ ID NO: 1, SEQ ID NO: 2, or a protein having at least 85% (e.g., 85%, 90%, 95%, 98%, 99%, or 100%) homology to any one of the sequences.

In some specific embodiments, the nucleic acid that has been subjected to codon optimization comprises the nucleotide sequence set forth in SEQ ID NO: 7 or a nucleotide sequence having at least 85% (e.g., 85%, 90%, 95%, 98%, 99%, or 100%) homology thereto.

In some preferred embodiments, the coding sequence of the nucleic acid vector is a coding sequence that has been further subjected to CpG removal. Changing the composition of the CpG island or the GC content of the coding region may influence the selection of codon usage preference. In some embodiments, at least 1, 2, 3, 4, 5, 6, 7, 8, 9, 10, 20, 30, 40, 50 CpGs may be removed compared to a coding sequence without CpG removal. In some embodiments, at least 10%, 20%, 30%, 40%, 50%, 60%, 70%, 80%, 90%, or 100% of CpG may be removed compared to a coding sequence without CpG removal. In other preferred embodiments, the coding sequence is a coding sequence that has been subjected to CpG removal and codon optimization. In some specific embodiments, the nucleic acid that has been subjected to codon optimization and CpG removal comprises the nucleotide sequence set forth in SEQ ID NO: 8 or a nucleotide sequence having at least 85% (e.g., 85%, 90%, 95%, 98%, 99%, or 100%) homology thereto.

In some preferred embodiments, the coding sequence encoding the anti-VEGF protein further comprises an intron insertion. In some preferred embodiments, the coding sequence encoding the anti-VEGF protein has been subjected to codon optimization, CpG removal, and intron insertion simultaneously. The intron in the coding sequence described in the present disclosure is selected from: a VH4 intron, an SV40 intron, a Chi intron, and a Chimeric intron. In some specific embodiments, the VH4 intron, the SV40 intron, the Chi intron, and the Chimeric intron have the nucleotide sequence set forth in SEQ ID NO: 3, SEQ ID NO: 4, SEQ ID NO: 5, or SEQ ID NO: 20, respectively. The present disclosure unexpectedly finds that the introduction of an intron at a specific position in the coding sequence encoding the anti-VEGF protein can effectively increase the expression level of the protein, such as by 10%, 20%, 30%, 40%, 50%, 60%, 70%, 80%, 90%, 100%, or more than 100%.

In a specific embodiment, the nucleic acid of the coding sequence that has been subjected to codon optimization, CpG removal, and VH4 intron insertion comprises the nucleotide sequence set forth in SEQ ID NO: 6 or SEQ ID NO: 13 or a nucleic acid sequence having at least 85% (e.g., 85%, 90%, 95%, 98%, 99%, or 100%) homology thereto. In another specific embodiment, the nucleic acid of the coding sequence that has been subjected to codon optimization, CpG removal, and SV40 intron insertion comprises the nucleotide sequence set forth in SEQ ID NO: 21 or a nucleic acid sequence having at least 85% (e.g., 85%, 90%, 95%, 98%, 99%, or 100%) homology thereto. In another specific embodiment, the nucleic acid of the coding sequence that has been subjected to codon optimization, CpG removal, and Chimeric intron insertion comprises the nucleotide sequence set forth in SEQ ID NO: 22 or a nucleic acid sequence having at least 85% (e.g., 85%, 90%, 95%, 98%, 99%, or 100%) homology thereto.

Therefore, another aspect of the present disclosure is to provide a polynucleotide expression cassette, wherein the polynucleotide expression cassette comprises the nucleic acid described in the present disclosure and an expression regulatory element (as described above, the nucleic acid described in the present disclosure comprises a coding sequence encoding an anti-VEGF protein).

For example, the expression regulatory element comprises a promoter, and the promoter is operably linked to the coding sequence of the nucleic acid molecule. A promoter includes a promoter sequence or a functional fragment thereof. The promoter is specific for eukaryotic cells or mammalian cells. The promoter is selected from the group consisting of: a β-actin (CBA) promoter, a cytomegalovirus (CMV) promoter, an elongation factor 1α (EF1α) promoter, a CAG promoter, an EFS promoter, an NA65P promoter, an MNT promoter, a UB6 promoter, a CAG promoter, an RPE65 promoter, and an opsin promoter.

The expression regulatory element of the present disclosure may further comprise an intron, and the intron is located downstream of the promoter. In some cases, an intron may refer to any sequence that can be transcribed but not translated. In some cases, an intron may refer to any sequence that is transcribed and removed from a mature RNA transcript in a cell. The intron is selected from a VH4 intron, an SV40 intron, a Chi intron, a chicken β-actin intron, a U12 intron, an RHD intron, and the like.

The expression regulatory element of the present disclosure may also comprise a polyadenylation signal (polyA). Polyadenylation signals protect mRNA from exonuclease attack and are extremely important for transcription termination, export of mRNA from the nucleus, and translation. The polyadenylation signal contains multiple consecutive adenosine monophosphates and usually contains AAUAAA repeats. The polyadenylation signal described in the present disclosure is located downstream of the coding sequence encoding the VEGF antagonist. In some embodiments, the polyadenylation signal includes simian vacuolating virus 40 (SV40), human growth hormone (HGH), bovine growth hormone (BGH), or β-globin (RGB).

In certain embodiments, the polynucleotide expression cassette of the present disclosure is flanked at the 5' and 3' ends by functional adenovirus inverted terminal repeats (ITRs). A functional adenovirus inverted terminal repeat (ITR) refers to an ITR sequence used to integrate, replicate, and package an AAV virion. The inverted terminal repeat is an adeno-associated virus ITR of a serotype selected from AAV1 ITR, AAV2 ITR, AAV3 ITR, AAV4 ITR, AAV5 ITR, AAV6 ITR, AAV7 ITR, AAV8 ITR, AAV9 ITR, AAV10 ITR, AAV11 ITR, and AAV12ITR.

In some embodiments, the polynucleotide expression cassette comprises, in a 5' to 3' order:
1) a 5' ITR;
2) a promoter;
3) an intron;
4) the aforementioned nucleic acid encoding the anti-VEGF protein of the present disclosure;
5) a polyadenylation signal sequence;
6)a3'ITR.

The promoter is selected from the group consisting of: a β-actin (CBA) promoter, a cytomegalovirus (CMV) promoter, an elongation factor 1α (EF1α) promoter, a UBC promoter, an EFS promoter, an NA65P promoter, an MNT promoter, a UB6 promoter, a CAG promoter, an RPE65 promoter, and an opsin promoter.

The intron is selected from a VH4 intron, an SV40 intron, a Chi intron, a chicken β-actin intron, a U12 intron, an RHD intron, and the like.

The polyadenylation signal includes simian vacuolating virus 40 (SV40), human growth hormone (HGH), bovine growth hormone (BGH), or β-globin (RGB).

In some more specific embodiments, the nucleic acid encoding the anti-VEGF protein encodes SEQ ID NO: 1 or SEQ ID NO: 2 or an amino acid sequence having at least 85% homology thereto.

In some more specific embodiments, the nucleic acid encoding the anti-VEGF protein comprises SEQ ID NO: 6, SEQ ID NO: 7, SEQ ID NO: 8, SEQ ID NO: 13, SEQ ID NO: 21, SEQ ID NO: 22, or a nucleotide sequence having at least 85% homology thereto.

In some more specific embodiments, the polynucleotide expression cassette described in the present disclosure comprises, in a 5' to 3' order:
1) a 5' ITR;
2) a CBA promoter;
3) a chicken β-actin intron;
4) a kozak sequence;
5) a nucleic acid encoding an anti-VEGF protein, wherein the nucleic acid encodes SEQ ID NO: 1 or SEQ ID NO: 2 or an amino acid sequence having at least 85% homology thereto;
6) an RGB polyadenylation signal sequence;
7)a3'ITR.

In some more specific embodiments, the polynucleotide expression cassette described in the present disclosure comprises, in a 5' to 3' order:
1) a 5' ITR;
2) a CBA promoter;
3) a chicken β-actin intron;
4) a kozak sequence;
5) a nucleic acid encoding an anti-VEGF protein, wherein the nucleic acid comprises SEQ ID NO: 6, SEQ ID NO: 7, SEQ ID NO: 8, SEQ ID NO: 13, SEQ ID NO: 21, SEQ ID NO: 22, or a nucleotide sequence having at least 85% homology thereto;
6) an RGB polyadenylation signal sequence;
7)a3'ITR.

In some more specific embodiments, the polynucleotide expression cassette described in the present disclosure comprises, in a 5' to 3' order:
1) a 5' ITR having the nucleotide sequence set forth in SEQ ID NO: 14;
2) a CBA promoter having the nucleotide sequence set forth in SEQ ID NO: 15;
3) a chicken β-actin intron having the nucleotide sequence set forth in SEQ ID NO: 16;
4) a kozak sequence having the nucleotide sequence set forth in SEQ ID NO: 17;
5) a nucleic acid encoding an anti-VEGF protein, wherein the nucleic acid comprises SEQ ID NO: 6, SEQ ID NO: 7, SEQ ID NO: 8, SEQ ID NO: 13, SEQ ID NO: 21, SEQ ID NO: 22, or a nucleotide sequence having at least 85% homology thereto;
6) an RGB polyadenylation signal sequence having the nucleotide sequence set forth in SEQ ID NO: 18;
7) a 3' ITR having the nucleotide sequence set forth in SEQ ID NO: 19.

In some specific embodiments, the nucleic acid encoding the anti-VEGF protein in the polynucleotide expression cassette has been subjected to codon optimization. In some more specific embodiments, the polynucleotide expression cassette (the nucleic acid encoding the anti-VEGF protein has been subjected to codon optimization) comprises, from 5' to 3', the nucleotide sequence set forth in SEQ ID NO: 27 or a sequence having at least 85% (e.g., 85%, 90%, 95%, 98%, 99%, or 100%) homology thereto.

In some specific embodiments, the nucleic acid encoding the anti-VEGF protein in the polynucleotide expression cassette has been subjected to CpG removal. In some more specific embodiments, the polynucleotide expression cassette (the nucleic acid encoding the anti-VEGF protein has been subjected to codon optimization and CpG removal) comprises, from 5' to 3', the nucleotide sequence set forth in SEQ ID NO: 29 or a sequence having at least 85% (e.g., 85%, 90%, 95%, 98%, 99%, or 100%) homology thereto.

In some specific embodiments, the nucleic acid encoding the anti-VEGF protein in the polynucleotide expression cassette further comprises an intron insertion. The intron illustratively includes, but is not limited to, a VH4 intron, an SV40 intron, a Chi intron, and a Chimeric intron. In some preferred embodiments, the nucleic acid encoding the anti-VEGF protein comprises an insertion of a VH4 intron, and the polynucleotide expression cassette comprises, from 5' to 3', the nucleotide sequence set forth in SEQ ID NO: 9 or SEQ ID NO: 31 or a nucleic acid sequence having at least 85% (e.g., 85%, 90%, 95%, 98%, 99%, or 100%) homology thereto. In some preferred embodiments, the nucleic acid encoding the anti-VEGF protein comprises an insertion of an SV40 intron, and the polynucleotide expression cassette comprises, from 5' to 3', the nucleotide sequence set forth in SEQ ID NO: 23 or a nucleic acid sequence having at least 85% homology thereto. In some preferred embodiments, the nucleic acid encoding the anti-VEGF protein comprises an insertion of a Chimeric intron, and the polynucleotide expression cassette comprises, from 5' to 3', the nucleotide sequence set forth in SEQ ID NO: 24 or a nucleic acid sequence having at least 85% (e.g., 85%, 90%, 95%, 98%, 99%, or 100%) homology thereto.

In some more specific embodiments, the polynucleotide expression cassette described in the present disclosure comprises, in a 5' to 3' order:
1) a 5' ITR;
2) a CBA promoter;
3) a chicken β-actin intron;
4) a kozak sequence;
5) a nucleic acid encoding an anti-VEGF protein, wherein the nucleic acid encodes SEQ ID NO: 1 or SEQ ID NO: 2 or an amino acid sequence having at least 85% homology thereto;
6) an SV40 polyadenylation signal sequence;
7)a3'ITR.

In some more specific embodiments, the polynucleotide expression cassette described in the present disclosure comprises, in a 5' to 3' order:
1) a 5' ITR;
2) a CBA promoter;
3) a chicken β-actin intron;
4) a kozak sequence;
5) a nucleic acid encoding an anti-VEGF protein, wherein the nucleic acid comprises SEQ ID NO: 6 or a nucleotide sequence having at least 85% homology thereto;
6) an SV40 polyadenylation signal sequence;
7)a3'ITR.

In some more specific embodiments, the polynucleotide expression cassette comprises, from 5' to 3', the nucleotide sequence set forth in SEQ ID NO: 33 or a sequence having at least 85% (e.g., 85%, 90%, 95%, 98%, 99%, or 100%) homology thereto.

Another aspect of the present disclosure is to provide a plasmid, wherein the plasmid comprises the aforementioned nucleic acid encoding the anti-VEGF protein or polynucleotide expression cassette of the present disclosure. In some preferred embodiments, the plasmid described in the present disclosure comprises the nucleotide sequence set forth in SEQ ID NO: 10, SEQ ID NO: 28, SEQ ID NO: 30, SEQ ID NO: 32, or SEQ ID NO: 34 or a sequence having at least 85% (e.g., 85%, 90%, 95%, 98%, 99%, or 100%) homology thereto.

The expression vector of the present disclosure described above shows an increased expression level of the anti-VEGF protein in target cells or target tissues. For example, after 2 µg of a single plasmid is transiently transfected into 293 cells, the protein expression level of the plasmid is increased by 10%, 20%, 30%, 40%, 50%, 60%, 70%, 80%, 90%, 100%, 150%, 200%, 300%, 400%, 500%, or more relative to that of an non-optimized plasmid.

For example, after 2.5 µg of a single plasmid is transiently transfected into APRE cells, the protein expression level of the plasmid is increased by 10%, 20%, 30%, 40%, 50%, 60%, 70%, 80%, 90%, 100%, 150%, 200%, 300%, 400%, 500%, or more relative to that of an non-optimized plasmid.

Another aspect of the present disclosure provides a recombinant virus, wherein the recombinant virus comprises: a capsid protein and the polynucleotide expression cassette described in the present disclosure. In some embodiments, the recombinant virus is a recombinant adeno-associated virus (rAAV), wherein the recombinant adeno-associated virus comprises an rAAV capsid protein and the polynucleotide cassette described in the present disclosure.

In some embodiments, the rAAV of the present disclosure comprises capsid proteins, which are structural proteins encoded by the cap genes of the AAV. The rAAV comprises three capsid proteins, referred to as VP1, VP2, and VP3, all of which are transcribed from a single cap gene via alternative splicing. VP1, VP2, and VP3 have molecular weights of about 87 kDa, about 72 kDa, and about 62 kDa, respectively. After translation, the capsid proteins form a spherical 60-mer protein shell around the viral genome. Capsid proteins function to protect the viral genome, deliver the genome, and interact with the host.

The capsid protein described in the present disclosure may be derived from any serotype of adeno-associated virus, including but not limited to AAV 1, AAV2, AAV3, AAV4, AAV5, AAV6, AAV7, AAV8, AAV9, AAV10, AAV11, AAV12, and the like. Any adeno-associated virus serotype of the adeno-associated virus serotypes can serve as a gene delivery vector. For example, the AAV capsid may be a wild-type capsid or a natural capsid. Wild-type AAV capsids of particular interest include AAV2, AAV5, AAV8, and AAV9. As with the ITRs, the capsid does not have to be a wild-type capsid, and modifications through nucleotide insertions, deletions, or substitutions may be introduced into the wild-type VP1, VP2, or VP3 sequences, provided that the resulting capsid retains the ability to transduce specific cells or tissues. In other words, the AAV capsid may be a variant AAV capsid, and the variant AAV capsid comprises one or more amino acid substitutions, deletions, or insertions relative to a parental capsid protein or an AAV capsid protein.

In some embodiments, the AAV capsid variant comprises a substitution, insertion, or deletion of about 1 to about 100 amino acids (e.g., between 1-10 amino acids, between 1-20 amino acids, between 1-30 amino acids, between 20-50 amino acids, between 20-60 amino acids, between 50-80 amino acids, between 50-100 amino acids, between 60-100 amino acids, etc.) compared to a known parental AAV capsid (e.g., AAV serotype 2, AAV2/3 (e.g., AAV2/3 hybrid), AAV serotype 8). In some embodiments, the AAV capsid variant comprises a substitution, insertion, or deletion of more than 100 amino acids (e.g., between 100-200 amino acids, between 200-300 amino acids, between 100-500 amino acids, between 500-1000 amino acids, or more) relative to a parental capsid. In some embodiments, the AAV capsid variant may comprise a substitution, insertion, or deletion of about 5 to about 50 amino acids (e.g., between 5-10 amino acids, between 5-20 amino acids, between 5-30 amino acids, between 5-40 amino acids, between 10-20 amino acids, between 10-30 amino acids, between 10-40 amino acids, between 10-50 amino acids, or between 30-50 amino acids) relative to a parental capsid (e.g., AAV serotype 2, AAV2/3 (e.g., AAV2/3 hybrid), AAV serotype 8). In some embodiments, the AAV capsid variant may comprise a substitution, insertion, or deletion of about 10 to about 30 (e.g., 10, 11, 12, 13, 14, 15, 16, 17, 18, 19, 20, 21, 22, 23, 24, 25, 26, 27, 28, 29, or 30) amino acids relative to a parental capsid (e.g., AAV serotype 2, AAV2/3 (e.g., AAV2/3 hybrid), AAV serotype 8). In some embodiments, the AAV capsid variant may comprise a substitution, insertion, or deletion of 1, 2, 3, 4, 5, 6, 7, 8, 9, 10, 11, 12, 13, 14, 15, 16, 17, 18, 19, or 20 amino acids relative to a parental capsid (e.g., AAV serotype 2, AAV2/3 (e.g., AAV2/3 hybrid), AAV serotype 8).

In some embodiments, the capsid variant may be a chimeric capsid variant. The chimeric capsid variant sequence may comprise portions of two or more AAV capsid serotypes or variants thereof. In some embodiments, the chimeric capsid comprises portions of 2, 3, 4, 5, 6, 7, 8, 9, 10, or more different capsid protein serotypes. In some embodiments, the chimeric capsid protein exhibits different properties, such as the tissue tropism possessed by AAV capsid proteins from which it is derived, and the like. Fragments can be incorporated by any appropriate method, e.g., recombinant DNA cloning.

In some embodiments, the AAV capsid variant described in the present disclosure is a variant of AAV2, AAV2/3 (e.g., AAV2/3 hybrid), or AAV8. AAV2 has been observed to efficiently transduce ocular tissues (e.g., photoreceptor cells and retinal pigment epithelium (RPE)), human central nervous system (CNS) tissues, kidney tissues, and other tissues. In some embodiments, the AAV capsid described in the present disclosure is an AAV2 variant, and the AAV2 variant may be used to deliver gene therapy to ocular tissues (e.g., the retina). AAV3 was observed to efficiently transform cancer cells. In some embodiments, the AAV variant described in the present disclosure is AAV2/3 (e.g., AAV2/3 hybrid). AAV8 has also been observed to transduce ocular tissues (e.g., retinal ganglion cells, stem cells).

In one embodiment, the modified AAV capsid protein is a modified AAV8 capsid protein. The applicant has studied and applied for a related patent CN202211691417.8 or PCT/CN2022/142185 (entitled MODIFIED AAV CAPSID PROTEIN AND USE THEREOF) of a capsid variant of AAV8, the entire contents of which are incorporated herein. In some preferred embodiments, the capsid protein is a modified AAV8 capsid protein, and the modified capsid protein comprises a heterologous polypeptide substitution of about 5-14 amino acids at amino acid positions 588-592 of the parental AAV8 (the amino acids at positions 588-592 of the parental/wild-type AAV8 capsid protein are "QQNTA"), such as a polypeptide sequence RGNQQNTARQ (SEQ ID NO: 11).

In some embodiments, the rAAV described in the present disclosure is a single-stranded AAV (ssAAV) or a double-stranded AAV (scAAV). The ssAAV refers to an rAAV having a coding sequence and a complementary sequence of a target gene expression cassette on separate strands and packaged in separate viral capsids. The scAAV is a double-stranded DNA designed from the coding region of the rAAV.

Another aspect of the present disclosure is to provide a host cell, wherein the host cell comprises the aforementioned nucleic acid encoding the anti-VEGF protein or polynucleotide expression cassette or plasmid of the present disclosure. The components to be cultured in the host cell to encapsulate the rAAV vector in the AAV capsid are reversely provided to the host cell. Any one or more of the required components (e.g., a recombinant AAV vector, a rep sequence, a cap sequence, and/or a helper function) may be provided by a stable host cell, and the host cell has been designed to contain one or more of the required components using methods known in the art. Preferably, stable host cells will contain the required components under the control of an inducible promoter. Alternative types of promoters of the present disclosure have been described in detail previously in the present disclosure.

"Host cell" refers to any cell that contains or is capable of containing a target substance. Generally, the host cell is a mammalian cell. In some embodiments, the host cell is a photoreceptor cell, a retinal pigment epithelial cell, a keratinocyte, a corneal cell, and/or a tumor cell. The host cell may serve as an AAV helper construct, an AAV vector, a helper function vector, or a receptor for other transferred DNA associated with the production of the recombinant AAV. The term includes the progeny of the original cell that has been transfected. Thus, as used herein, "host cell" may refer to a cell that has been transfected with an exogenous DNA sequence. It can be understood that the progeny of a single parent cell may not necessarily be completely identical in morphology, or in genome, or in total DNA complement to the original parent, due to natural, accidental, or deliberate mutation. In some embodiments, the host cell is a mammalian cell, a yeast cell, a bacterial cell, an insect cell, a plant cell, or a fungal cell. In some embodiments, the host cell is a neuron, a photoreceptor cell, a pigmented retinal epithelial cell, or a glial cell. The recombinant AAV vector, the rep sequence, the cap sequence, and the helper function required to produce the rAAV of the present disclosure can be delivered to a packaging host cell using any appropriate genetic element (vector). The genetic element can be delivered by any suitable method known to those skilled in the art, such as genetic engineering, recombinant engineering, and synthetic techniques.

### Pharmaceutical composition

Another aspect of the present disclosure is to provide a pharmaceutical composition. The pharmaceutical composition comprises the polynucleotide expression cassette, the plasmid, or the recombinant virus of the present disclosure, and a pharmaceutically acceptable excipient, carrier, or diluent.

Those skilled in the art can readily select suitable diluents, carriers, or excipients. As used herein, carriers or excipients include any solvent, dispersion medium, vehicle, coating, diluent, antibacterial agent and antifungal agent, isotonic agent, absorption delaying agent, buffer, carrier solution, suspension, colloid, preservative, chemical stabilizer, or the like.

Exemplary carriers or excipients include sterile normal saline, lactose, sucrose, calcium phosphate, gelatin, dextran, agar, pectin, peanut oil, sesame oil, and water. For example, one suitable excipient or carrier includes saline, which may be formulated with a variety of buffer solutions (e.g., phosphate buffer). The action of microorganisms can be prevented by various antibacterial and antifungal agents, such as parabens, chlorobutanol, phenol, sorbic acid, and thimerosal. In many cases, it will be preferable to include isotonic agents, for example, sugars or sodium chloride. The absorption of injectable compositions can be prolonged by the use in the compositions of agents that delay absorption, such as aluminum monostearate and gelatin.

To administer injectable aqueous solutions, buffer solutions may be suitably formulated as necessary, and the liquid diluent is first rendered isotonic with sufficient saline or glucose. These specific aqueous solutions are particularly suitable for intravenous, intramuscular, subcutaneous, and intraperitoneal administration.

Sterile injectable solutions are prepared by mixing a required amount of an active rAAV with various other ingredients enumerated in the present disclosure in an appropriate solvent, followed by filtration and sterilization. Generally, dispersions are prepared by incorporating the various sterilized active ingredients into a sterile vehicle that contains a dispersion medium and other required ingredients.

The rAAV composition of the present disclosure may also be formulated in a neutral or salt form. Pharmaceutically acceptable salts include acid addition salts and salts formed with inorganic acids (e.g., hydrochloric acid or phosphoric acid) or organic acids (e.g., acetic acid, oxalic acid, tartaric acid, mandelic acid). Salts formed with free carboxyl groups may also be derived from inorganic bases (e.g., sodium, potassium, ammonium, calcium, or ferric hydroxide) or organic bases (e.g., isopropylamine, trimethylamine, histidine, procaine, etc.). After the completion of formulation, the solution will be administered in a manner compatible with the dosage form and in a therapeutically effective amount. The formulation is readily administered in a variety of dosage forms, such as an injectable solution and a drug-release capsule.

The composition of the present disclosure can be delivered into a suitable host cell via a delivery carrier such as a liposome, a nanocapsule, a microparticle, a microsphere, a lipid particle, and a vesicle.

It is advantageous to formulate injectable, oral, or parenteral compositions in unit dosage form for ease of administration. As used herein, the unit dosage form refers to a physically discrete unit suitable as a single dose for the subject to be treated; each unit contains a predetermined amount of active compound calculated to produce the desired therapeutic effect in association with the required pharmaceutical carrier. The specification of the unit dosage form of the present disclosure is determined by the unique characteristics of the active compound and the specific therapeutic effect to be achieved, as well as the limitations inherent in the art of compounding such an active compound for the treatment of an individual. For example, a unit dose may be a certain amount of vector genomes, or a certain amount of vector genomes per milliliter, or a unit dose of the pharmaceutical composition measured using the multiplicity of infection (MOI), where MOI refers to the ratio or multiple of a vector or viral genome to a cell to which a nucleic acid can be delivered. The pharmaceutical composition may be contained in a container, package, or dispenser (e.g., a syringe) together with instructions for administration.

In some embodiments, the pharmaceutical composition is prepared as a formulation suitable for intravitreal injection, subretinal injection, suprachoroidal space injection, intravenous injection, intratumoral injection, or intramuscular injection.

### Delivery of recombinant adeno-associated virus (rAAV)

Another aspect of the present disclosure is to provide use of the polynucleotide expression cassette, the plasmid, or the recombinant virus described in the present disclosure in the manufacture of a medicament for treating a VEGF-associated disease. Alternatively, another aspect of the present disclosure is to provide a method for treating a VEGF-associated disease in a mammalian subject, wherein the method comprises delivering a therapeutically effective amount of the polynucleotide expression cassette, the plasmid, or the recombinant virus described in the present disclosure to the eye.

The rAAV described in the present disclosure may be delivered to a subject according to any suitable method known in the art. For example, the rAAV suspended in a physiologically compatible carrier (e.g., in a composition) is preferably administered to a subject, i.e., a host animal, such as a human, mouse, rat, cat, dog, sheep, rabbit, horse, cow, goat, pig, guinea pig, hamster, chicken, turkey, or non-human primate (e.g., a macaque). In some embodiments, the host animal does not include a human. In some embodiments, the subject is a human.

"Therapeutically effective amount" refers to an amount that is effective, at the dosage and for the duration required, to achieve the desired therapeutic effect. A therapeutically effective amount of the rAAV virus or the pharmaceutical composition may vary depending on factors such as the disease state, age, sex, and body weight of the subject to be treated, as well as the ability of the rAAV virus or pharmaceutical composition to elicit the desired response in the subject. The administration regimen can be adjusted to provide the optimum therapeutic response. Generally, a therapeutically effective amount is also one in which any toxic or detrimental effects of the rAAV virus or the pharmaceutical composition are outweighed by the therapeutically beneficial effects. "Prophylactically effective amount" refers to an amount that is effective, at the dosage and for the duration required, to achieve the desired prophylactic effect, such as preventing or inhibiting various disorders. A prophylactic dose may be used in subjects before the onset of a disease or in its early stage, and in some cases, the prophylactically effective amount may be greater or smaller than the therapeutically effective amount. The administration dose depends largely on the condition and size of the subject being treated, as well as the formulation for treatment, frequency of treatment, and route of administration. The regimen for sustained treatment, including dosage, formulation, and frequency, may be guided by the initial response and clinical judgment.

In some embodiments, the recombinant adeno-associated virus is administered at a dose of E8/eye to E16/eye. In some preferred embodiments, the recombinant adeno-associated virus is administered at a dose of E8/eye to E15/eye. In some preferred embodiments, the recombinant adeno-associated virus is administered at a dose of E9/eye to E15/eye. In some preferred embodiments, the recombinant adeno-associated virus is administered at a dose of E9/eye to E14/eye. In other preferred embodiments, the recombinant adeno-associated virus is administered at a dose of E10/eye to E14/eye. In other preferred embodiments, the recombinant adeno-associated virus is administered at a dose of E11/eye to E14/eye. In other preferred embodiments, the recombinant adeno-associated virus is administered at a dose of E12/eye to E14/eye. In other preferred embodiments, the recombinant adeno-associated virus is administered at a dose of E13/eye to E14/eye. In other preferred embodiments, the recombinant adeno-associated virus is administered at a dose of E8/eye to E13/eye. In other preferred embodiments, the recombinant adeno-associated virus is administered at a dose of E9/eye to E13/eye. In other preferred embodiments, the recombinant adeno-associated virus is administered at a dose of E10/eye to E13/eye. In other preferred embodiments, the recombinant adeno-associated virus is administered at a dose of E11/eye to E13/eye. In other preferred embodiments, the recombinant adeno-associated virus is administered at a dose of E12/eye to E13/eye. In other preferred embodiments, the recombinant adeno-associated virus is administered at a dose of E8/eye to E12/eye. In other preferred embodiments, the recombinant adeno-associated virus is administered at a dose of E9/eye to E12/eye. In other preferred embodiments, the recombinant adeno-associated virus is administered at a dose of E10/eye to E12/eye. In other preferred embodiments, the recombinant adeno-associated virus is administered at a dose of E11/eye to E12/eye. In other preferred embodiments, the recombinant adeno-associated virus is administered at a dose of E8/eye to E11/eye. In other preferred embodiments, the recombinant adeno-associated virus is administered at a dose of E9/eye to E11/eye. In other preferred embodiments, the recombinant adeno-associated virus is administered at a dose of E10/eye to E11/eye. In other preferred embodiments, the recombinant adeno-associated virus is administered at a dose of E8/eye to E10/eye. In other preferred embodiments, the recombinant adeno-associated virus is administered at a dose of E9/eye to E10/eye. In other specific embodiments, the recombinant adeno-associated virus is administered at a dose of E8/eye, 2E8/eye, 3E8/eye, 4E8/eye, 5E8/eye, 6E8/eye, 7E8/eye, 8E8/eye, 9E8/eye, E9/eye, 2E9/eye, 3E9/eye, 4E9/eye, 5E9/eye, 6E9/eye, 7E9/eye, 8E9/eye, 9E9/eye, E10/eye, 2E10/eye, 3E10/eye, 4E10/eye, 5E10/eye, 6E10/eye, 7E10/eye, 8E10/eye, 9E10/eye, E11/eye, 2E11/eye, 3E11/eye, 4E11/eye, 5E11/eye, 6E11/eye, 7E11/eye, 8E11/eye, 9E11/eye, E12/eye, 2E12/eye, 3E12/eye, 4E12/eye, 5E12/eye, 6E12/eye, 7E12/eye, 8E12/eye, 9E12/eye, E13/eye, 2E13/eye, 3E13/eye, 4E13/eye, 5E13/eye, 6E13/eye, 7E13/eye, 8E13/eye, 9E13/eye, E14/eye, 2E14/eye, 3E14/eye, 4E14/eye, 5E14/eye, 6E14/eye, 7E14/eye, 8E14/eye, or 9E14/eye. In some preferred embodiments, the recombinant adeno-associated virus is administered at a dose of 5E11/eye to 2E12/eye. In some more preferred embodiments, the recombinant adeno-associated virus is administered at a dose of 2E12/eye.

In some embodiments, the rAAV virus or the pharmaceutical composition is administered to a subject once daily, once weekly, once every two weeks, once monthly, once every 2 months, once every 3 months, once every 6 months, once yearly or once every 2 years, once every 5 years, or once a lifetime.

Exemplary routes of administration and delivery include intravenous (I.V.), intraarticular, intraperitoneal (I.P.), intra-arterial, intramuscular, parenteral, subcutaneous, intrapleural, dermal, transdermal, parenteral (e.g., transmucosal), intracranial, intraspinal, oral (digestive), mucosal, respiratory, intranasal, intubation, intrapulmonary, intrapulmonary instillation, buccal, sublingual, intravascular, intrathecal, intracavitary, iontophoretic, intraocular, intraglandular, intraorgan, and intrafallopian routes.

In some embodiments, the rAAV may be delivered to a mammalian subject via, for example, intraocular injection, subretinal injection, choroidal injection (e.g., suprachoroidal space injection), or regional administration (e.g., eye drops), or by injection into an ocular tissue to affect the eyes of the mammal (e.g., intravitreal injection). "Ocular tissue" refers to any tissue derived from or contained in the eye. Non-limiting examples of ocular tissues include neurons, the retina (e.g., photoreceptor cells), the sclera, the choroid, the retina, the vitreous body, the macula, the fovea, the optic disc, the lens, the pupil, the iris, the aqueous humor, the cornea, the conjunctiva, the ciliary body, and the optic nerve. The retina is located at the back of the eye and is composed of photoreceptor cells. These photoreceptor cells (e.g., rods, cones) enable vision by discerning color and contrast in the visual field. In some embodiments, the rAAV or the composition described in the present disclosure is administered via intraocular injection. In some embodiments, the rAAV or the composition described in the present disclosure is administered via intravitreal injection. In some embodiments, the rAAV or the composition described in the present disclosure is administered via subretinal injection. In some embodiments, the rAAV or the composition described in the present disclosure is administered via intrachoroidal (e.g., suprachoroidal space) injection. In some embodiments, the rAAV or the composition described in the present disclosure is administered via intravenous injection. In some embodiments, the recombinant virus or the pharmaceutical composition described in the present disclosure is administered via intravitreal injection, subretinal injection, suprachoroidal space injection, intravenous injection, intratumoral injection, or intramuscular injection.

"VEGF-associated disease" refers to a group of diseases associated with abnormal VEGF activity/signaling. Numerous studies have confirmed that abnormally excessive VEGF can stimulate and induce pathological angiogenesis, leading to angiogenesis-related ocular diseases. Non-limiting exemplary angiogenesis-related ocular diseases include angiogenesis-dependent cancers, angiogenesis-related ocular diseases, solid tumors (e.g., lung cancer, breast cancer, kidney cancer, liver cancer, pancreatic cancer, head and neck cancer, colon cancer), melanoma, hematologic tumors (e.g., leukemia), metastatic tumors, benign tumors (e.g., hemangioma, acoustic neuroma, neurofibroma, tracheitis, and pyogenic granuloma), rheumatoid arthritis, psoriasis, redness and swelling, Osier-Webber syndrome, myocardial angiogenesis, plaques, telangiectasia, hemophilic arthropathy, or angiofibroma.

In some embodiments, angiogenesis-related ocular diseases include, but are not limited to, diabetic retinopathy, retinopathy of prematurity, macular degeneration, corneal graft rejection, neovascular glaucoma and retrolental fibroplasia, epidemic keratoconjunctivitis, vitamin A deficiency, contact lens overwear, atopic keratitis, bacterial keratitis, ulcers, ulcers, bacterial keratitis, ulcers, primary keratosis, rheumatoid arthritis, systemic lupus, polyarteritis, trauma, Wegener's sarcoidosis, scleritis, Stevens-Johnson syndrome, pemphigoid, radial keratotomy, corneal transplant rejection, sickle cell anemia, sarcoidosis, pseudoxanthoma elasticum, Paget's disease, vein occlusion, arterial occlusion, carotid occlusive disease, chronic uveitis/vitritis, mycobacterial infection, Lyme disease, systemic lupus erythematosus, retinopathy of prematurity, Eales disease, Behcet's disease, retinitis or choroiditis infection, presumed ocular histoplasmosis, Bests disease, myopia, fovea, Stargardt disease, pars planitis, chronic retinal detachment, hyperviscosity syndrome, toxoplasmosis, and post-traumatic or post-laser complications.

In some embodiments, the ocular disease is selected from retinal neovascularization, choroidal neovascularization, iris neovascularization, corneal neovascular ocular disease, non-infectious uveitis, or glaucoma.

In some embodiments, the ocular disease is selected from age-related macular degeneration, macular edema, diabetic macular edema, macular edema secondary to retinal vein occlusion, retinal vein occlusion, central retinal vein occlusion, branch retinal vein occlusion, macular edema caused by branch retinal vein occlusion, diabetic retinal edema, diabetic retinopathy, proliferative diabetic retinopathy, diabetic retinal ischemia, polypoidal choroidal vasculopathy, choroidal neovascularization secondary to degenerative myopia, or retinopathy of prematurity. Specific forms of macular degeneration may include acute macular degeneration, nonexudative age-related macular degeneration, and exudative age-related macular degeneration.

In some preferred embodiments, the ocular disease is selected from age-related macular degeneration, diabetic retinopathy, diabetic macular edema, central retinal vein occlusion, macular edema caused by branch retinal vein occlusion, macular edema secondary to retinal vein occlusion, polypoidal choroidal vasculopathy, wet age-related macular degeneration with very low vision, and choroidal neovascularization secondary to pathological myopia.

### DRAWINGS OF THE SPECIFICATION

FIG. 1 shows the schematic diagrams of polynucleotide expression cassettes in plasmids No. 3, No. 4, and No. 5.
FIG. 2 shows the grade 4 laser spot diagrams of the buffer group and the high-dose group in Example 4.

### DETAILED DESCRIPTION OF THE PREFERRED EMBODIMENTS

The present disclosure will be further illustrated with reference to the following specific examples. It should be understood that these examples are merely intended to illustrate the present disclosure rather than limit the scope of the present disclosure. Moreover, the following examples do not represent that the following experiments are all or the only experiments of the present disclosure.

### Example 1: Optimization of Anti-VEGF Protein Coding Sequence and Plasmid Construction

The primary objective of this example was to construct plasmids capable of expressing the anti-VEGF protein set forth in SEQ ID NO: 1. A series of polynucleotide expression cassettes comprising various combinations of regulatory elements and coding sequences (Table 1) were constructed using standard recombinant DNA cloning techniques or universal molecular biology techniques. Recombinant plasmids comprising the polynucleotide expression cassettes described in Table 1 were then constructed and cloned into *Escherichia coli* using conventional DNA recombination and cloning techniques.

The process first included optimization of the anti-VEGF protein coding sequence, involving, for example, codon optimization, CpG removal, and/or intron insertion. For example, in terms of codon optimization, a Homo sapiens codon optimization strategy was employed, whereby the CDS region was adapted to humanization preferences, GC content was reduced, and repetitive sequences were minimized. In terms of CpG removal optimization, the codon-optimized CDS region was further processed by removing CpG based on the preferences of the humanized codon optimization strategy. In terms of VH4 intron insertion, 2 insertion modes were designed based on the features of the amino acid primary and secondary structures, as well as the features of VH4 intron insertion, conforming to the precursor mRNA sequence signature "A/C A G G U...A G G", where the first 3 bases are exons, positions 3 and 4 constitute the 5' splice site, and the region between the penultimate and the last positions at the 3' end constitutes the 3' splice site.

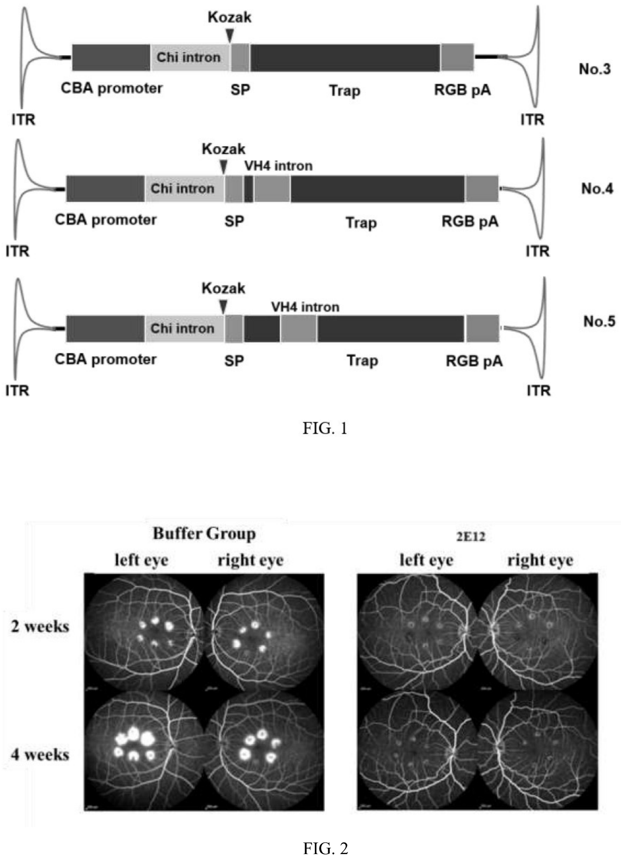

Subsequently, polynucleotide expression cassettes and plasmids based on the optimized or non-optimized anti-VEGF protein coding sequences described above were constructed, respectively. Exemplarily, the expression cassettes or plasmids No. 1-7 comprise, in a 5' to 3' order: an AAV 5' ITR, a CBA promoter, a chicken β-actin intron (denoted as Chi intron in FIG. 1), a Kozak sequence, a nucleic acid of the coding sequence encoding the anti-VEGF protein, a rabbit-globin (RGB) polyadenylation signal, and an AAV 5' ITR.

In the plasmids comprising the polynucleotide expression cassettes described above, the plasmid, in which the coding sequence of the anti-VEGF protein was not optimized, was designated as No. 1, wherein the nucleic acid of the coding sequence of the anti-VEGF protein is set forth in SEQ ID NO: 12, the nucleotide sequence of 5' to 3' is set forth in SEQ ID NO: 25, and the plasmid nucleotide sequence is set forth in SEQ ID NO: 26; the plasmid, in which the coding sequence of the anti-VEGF protein had been subjected to codon optimization, was designated as No. 2, wherein the nucleic acid of the coding sequence of the anti-VEGF protein is set forth in SEQ ID NO: 7, the nucleotide sequence of 5' to 3' is set forth in SEQ ID NO: 27, and the plasmid nucleotide sequence is set forth in SEQ ID NO: 28; the plasmid, in which the coding sequence of the anti-VEGF protein had been subjected to codon optimization and CpG removal, was designated as No. 3, wherein the nucleic acid of the coding sequence of the anti-VEGF protein is set forth in SEQ ID NO: 8, the nucleotide sequence of 5' to 3' is set forth in SEQ ID NO: 29, and the plasmid nucleotide sequence is set forth in SEQ ID NO: 30; in plasmids No. 4 and No. 5, VH4 introns were inserted at different positions in the coding sequences of the anti-VEGF protein, respectively, wherein in plasmid No. 4, the nucleic acid of the coding sequence of the anti-VEGF protein is set forth in SEQ ID NO: 6, the nucleotide sequence of 5' to 3' is set forth in SEQ ID NO: 9, and the plasmid nucleotide sequence is set forth in SEQ ID NO: 10; in plasmid No. 5, the nucleic acid of the coding sequence of the anti-VEGF protein is set forth in SEQ ID NO: 13, the nucleotide sequence of 5' to 3' is set forth in SEQ ID NO: 31, and the plasmid nucleotide sequence is set forth in SEQ ID NO: 32; in plasmids No. 6 and No. 7, an SV40 intron and a Chimeric intron were inserted into the coding sequences of the anti-VEGF protein, respectively; plasmids No. 8-19 contain different expression regulatory elements (e.g., a promoter, an intron, and a polyadenylation signal), wherein in plasmid No. 8, the nucleic acid of the coding sequence of the anti-VEGF protein is set forth in SEQ ID NO: 6, the nucleotide sequence of 5' to 3' is set forth in SEQ ID NO: 33, and the plasmid nucleotide sequence is set forth in SEQ ID NO: 34. The numbers and main compositions of the plasmids are shown in Table 1 below, and the main components of representative plasmids are shown in FIG. 1.

**Table 1**

| No. | Promoter | Intron | kozak | Coding sequence of target gene | polyadenylation signal |
|---|---|---|---|---|---|
| No.1 | CBA | chicken β-action | kozak | SEQ ID NO:12 | RGB |
| No.2 | CBA | chicken β-action | kozak | SEQ ID NO:7 | RGB |
| No.3 | CBA | chicken β-action | kozak | SEQ ID NO:8 | RGB |
| No.4 | CBA | chicken β-action | kozak | SEQ ID NO:6 | RGB |
| No.5 | CBA | chicken β-action | kozak | SEQ ID NO:13 | RGB |
| No.6 | CBA | chicken β-action | kozak | SEQ ID NO:21 | RGB |
| No.7 | CBA | chicken β-action | kozak | SEQ ID NO:22 | RGB |
| No.8 | CBA | chicken β-action | kozak | SEQ ID NO:6 | SV40 |
| No.9 | CBA | chicken β-action | kozak | SEQ ID NO:6 | BGH |
| No.10 | CBA | chicken β-action | kozak | SEQ ID NO:6 | HGH |
| No.11 | CMV | chicken β-action | kozak | SEQ ID NO:6 | RGB |
| No.12 | EF1α | chicken β-action | kozak | SEQ ID NO:6 | RGB |
| No.13 | CAG | chicken β-action | kozak | SEQ ID NO:6 | RGB |
| No.14 | CBA | Chi | kozak | SEQ ID NO:6 | RGB |
| No.15 | CBA | PI | kozak | SEQ ID NO:6 | RGB |
| No.16 | CBA | SV40 | kozak | SEQ ID NO:6 | RGB |
| No.17 | CBA | chicken β-action | kozak | SEQ ID NO:6 | SV40 |
| No.18 | CBA | chicken β-action | kozak | SEQ ID NO:6 | BGH |
| No.19 | CBA | chicken β-action | kozak | SEQ ID NO:6 | HGH |

### Example 2: Protein Expression in 293 Cells Transiently Transfected with Plasmids

To evaluate the expression properties of each polynucleotide expression cassette or plasmid *in vitro,* each recombinant construct was transfected into suspended HEK293F cells using a transfection reagent. The specific method was as follows: A proper cell passage number (not exceeding P15) was selected; 2 h before plasmid transfection, 10 mL of DMEM medium (containing 10% FBS) was carefully added without interfering with the cell monolayer; then single plasmid transfection was performed via the Lipofectamine^{™} 2000 method, which specifically included: adding a plasmid containing 2 µg of target gene (endotoxin removed) to 1 mL of serum-free medium, and adding 5 µL of LiP2000 to another 1 mL of serum-free medium; allowing each mixture to stand for 5 min; then uniformly mixing the mixtures together gently, and allowing the resulting mixture to stand for 15 min; adding the mixture solution obtained from the previous step to the cell culture dish, distributing the mixture solution evenly across various positions during addition, and upon completion, uniformly mixing the entire culture medium gently, followed by culturing at 37 °C with 5% CO2; after 6 h of transfection, carefully pipetting off the medium, which was replaced with a fresh medium (10% FBS, 1% P/S), and culturing for another 48-72 h, followed by the collection of the cells and the cell supernatant; preparing cell lysates containing the expressed protein by 3 freeze-thaw cycles, and determining the protein content by ELISA (n = 3), with the results shown in Table 2.

**Table 2**

| Plasmid No. | Expression level in 293 cells by single plasmid transient transfection (ng/mL) |
|---|---|
| No.1 | 627.7 |
| No.2 | 1030.1 |
| No.3 | 719.1 |
| No.4 | 3810.3 |
| No.5 | 712.6 |

### Example 3: Protein Expression in APRE19 Cells Transiently Transfected with Plasmids

Each recombinant construct was transfected into suspended APRE19 cells using a transfection reagent. The specific method was as follows: A proper cell passage number (not exceeding P15) was selected; 2 h before plasmid transfection, 10 mL of DMEM medium (containing 10% FBS) was carefully added without interfering with the cell monolayer; then single plasmid transfection was performed via the Lipofectamine^{™} 2000 method, which specifically included: adding a plasmid containing 2.5 µg of target gene (endotoxin removed) to 1 mL of serum-free medium, and adding 5 µL of LiP2000 to another 1 mL of serum-free medium; allowing each mixture to stand for 5 min; then uniformly mixing the mixtures together gently, and allowing the resulting mixture to stand for 15 min; adding the mixture solution obtained from the previous step to the cell culture dish, distributing the mixture solution evenly across various positions during addition, and upon completion, uniformly mixing the entire culture medium gently, followed by culturing at 37 °C with 5% CO2; after 6 h of transfection, carefully pipetting off the medium, which was replaced with a fresh medium (10% FBS, 1% P/S), and culturing for another 48-72 h, followed by the collection of the cells and the cell supernatant; preparing cell lysates containing the expressed protein by 3 freeze-thaw cycles, and determining the protein content by ELISA (n = 3), with the results shown in Table 3.

**Table 3**

| Plasmid No. | Expression level in APRE19 cells by single plasmid transient transfection (ng/mL) |
|---|---|
| No.1 | 230.67 |
| No.4 | 946.87 |
| No.5 | 877.50 |
| No.8 | 498.83 |
| No.9 | 54.93 |
| No.10 | 86.30 |

### Example 3: Protein Expression in 293 Cells Infected with AAV Virus

The AAV capsid used in this example was a modified AAV8 capsid protein comprising a substitution at amino acid positions 588-592 of the parental AAV8 with the polypeptide sequence set forth in SEQ ID NO: 11 (RGNQQNTARQ). The construction of a plasmid containing the capsid protein is specifically described in the related patent PCT/CN2022/142185 (entitled MODIFIED AAV CAPSID PROTEIN AND USE THEREOF), which is incorporated herein.

AAV viruses were packaged via triple plasmid co-transfection into cells to generate: a first plasmid comprising the anti-VEGF protein coding sequence flanked by ITRs constructed in Example 1 (plasmid No. 4); a plasmid encoding the Rep/Cap genes as described above (a second plasmid, encoding the modified AAV8 capsid protein described above); and a third plasmid containing an adenovirus helper function gene. After transfection, the cells were collected and lysed to release the viruses, and the viral titer was determined by PCR for later use.

ARPE19 cells were seeded into a 6-well plate and cultured until the cell confluence was 70-80%; the medium (10% FBS, 1% P/S) was pipetted off and replaced with a serum-free and double antibody-free medium per well; the virus was added at the desired ratio (6E10 vg of AAV virus was added to 3.6E5 ARPE19 cells), such that the total volume of virus + serum-free and double antibody-free medium was 1000 µL; after 6 h of infection, 2 mL of fresh medium (10% FBS, 1% P/S) was added to each well, and the culture was continued until 72 h, after which the expression level of the target protein was detected by ELISA. The results indicated that the protein expression level of the AAV virus was 3437.6 ng/mL.

### Example 4: Efficacy Experiment in NHP Following AAV Virus Infection

### Animal model

A total of 16 cynomolgus monkeys were assigned to 4 groups: 1 negative control group and 3 dose-level test article groups (4 cynomolgus monkeys per group).

### Test drug

rAAV dose groups: Low-dose group - 5E11 vg/eye, medium-dose group - 1E12 vg/eye, high-dose group - 2E12 vg/eye (the rAAV was prepared according to Example 3 and dialyzed in a buffer (20 mM Tris-Cl pH 8.0, 150 mM NaCl, 1 mM MgCl₂, 0.003% Poloxamer 188)).

Negative control group (Vehicle): Buffer (20 mM Tris-Cl pH 8.0, 150 mM NaCl, 1 mM MgCl2, 0.003% Poloxamer 188).

### Method of administration

Administration was performed on day 0. The animals in the dose groups and the negative control group all received suprachoroidal space administration via injection in both eyes at 100 µL/eye. On day 21, each group of monkeys was subjected to laser modeling, with 6 laser modeling points for each eye (48 laser spots for each of the negative control group and the medium/low-dose groups, and no statistics were collected for one eye of a monkey in the high-dose group due to hemorrhage, resulting in 42 laser spots). On day 35 and day 49, i.e., 14 days and 28 days after modeling, respectively, the formation of grade 4 laser spots was assessed by FFA (fundus fluorescein angiography).

### Detection results

As shown in FIG. 2, 14 days after modeling, the number of grade 4 laser spots for the buffer group was 21; the number of grade 4 laser spots for the rAAV low-dose group was 16, the number of grade 4 laser spots for the rAAV medium-dose group was 11, and the number of grade 4 laser spots for the rAAV high-dose group was 0. 28 days after modeling, the number of grade 4 laser spots for the buffer group was 34, the number of grade 4 laser spots for the rAAV low-dose group was 20, the number of grade 4 laser spots for the rAAV medium-dose group was 12, and the number of grade 4 laser spots for the rAAV high-dose group was 0. The proportion of grade 4 laser spots for each group is shown in Table 4. The examples of FFA results for the rAAV high-dose group are shown in FIG. 2.

**Table 4**

| Groups | 2 weeks | 4 weeks |
|---|---|---|
| Buffer | 21/48 | 34/48 |
| 5E11 | 16/48 | 20/48 |
| 1E12 | 11/48 | 12/48 |
| 2E12 | 0/42 | 0/42 |

## Claims

1. A nucleic acid, wherein the nucleic acid comprises a coding sequence encoding an anti-VEGF protein, and the coding sequence has been subjected to codon optimization.

2. The nucleic acid according to claim 1, wherein the anti-VEGF protein comprises extracellular domain 2 of VEGF receptor 1 and extracellular domain 3 of VEGF receptor 2; preferably, the anti-VEGF protein further comprises extracellular domain 4 of VEGF receptor 2; more preferably, the anti-VEGF protein further comprises an immunoglobulin Fc fragment; more preferably, the anti-VEGF protein comprises SEQ ID NO: 1, SEQ ID NO: 2, or a protein having at least 85% homology to either of the sequences.

3. The nucleic acid according to claim 2, wherein the nucleic acid comprises SEQ ID NO: 7 or a sequence having at least 85% homology thereto.

4. The nucleic acid according to claim 1, wherein the coding sequence of the nucleic acid has been further subjected to CpG removal.

5. The nucleic acid according to claim 4, wherein the nucleic acid comprises SEQ ID NO: 8 or a sequence having at least 85% homology thereto.

6. The nucleic acid according to any one of claims 1-5, wherein the coding sequence of the nucleic acid further comprises an intron insertion.

7. The nucleic acid according to claim 6, wherein the intron is selected from a VH4 intron, an SV40 intron, a Chi intron, and a Chimeric intron; preferably, the VH4 intron, the SV40 intron, the Chi intron, or the Chimeric intron have the nucleotide sequence set forth in SEQ ID NO: 3, SEQ ID NO: 4, SEQ ID NO: 5, or SEQ ID NO: 20, respectively.

8. The nucleic acid according to claim 7, wherein the nucleic acid comprises the nucleotide sequence set forth in SEQ ID NO: 6, SEQ ID NO: 13, SEQ ID NO: 21, or SEQ ID NO: 22 or a nucleic acid sequence having at least 85% homology thereto.

9. A polynucleotide expression cassette, wherein the polynucleotide expression cassette comprises the nucleic acid according to any one of claims 1-8 and an expression regulatory element.

10. The polynucleotide expression cassette according to claim 9, wherein the polynucleotide expression cassette described in the present disclosure comprises, in a 5' to 3' order:
1) a 5' ITR;
2) a promoter;
3) an intron;
4) the nucleic acid according to any one of claims 1-8;
5) a polyadenylation signal sequence;
6) a 3' ITR.

11. The polynucleotide expression cassette according to claim 10, wherein the polynucleotide expression cassette comprises, in a 5' to 3' order:
1) a 5' ITR;
2) a CBA promoter;
3) a chicken β-actin intron;
4) a kozak sequence;
5) the nucleic acid according to any one of claims 1-8;
6) an RGB polyadenylation signal sequence;
7)a3'ITR; or
wherein the polynucleotide expression cassette comprises, in a 5' to 3' order:
1) a 5' ITR;
2) a CBA promoter;
3) a chicken β-actin intron;
4) a kozak sequence;
5) the nucleic acid according to any one of claims 1-8;
6) an SV40 polyadenylation signal sequence;
7)a3'ITR.

12. The polynucleotide expression cassette according to claim 11, wherein the polynucleotide expression cassette comprises the nucleotide sequence set forth in SEQ ID NO: 9, SEQ ID NO: 23, SEQ ID NO: 24, or SEQ ID NO: 27, SEQ ID NO: 29, SEQ ID NO: 31 or SEQ ID NO: 33 or a sequence having at least 85% homology thereto.

13. A plasmid, wherein the plasmid comprises the nucleic acid according to any one of claims 1-8 or the polynucleotide expression cassette according to any one of claims 9-12.

14. The plasmid according to claim 13, wherein the plasmid comprises the nucleotide sequence set forth in SEQ ID NO: 10, SEQ ID NO: 28, SEQ ID NO: 30, SEQ ID NO: 32, or SEQ ID NO: 34 or a sequence having at least 85% homology thereto.

15. A recombinant adeno-associated virus (rAAV), which comprises a capsid protein and the polynucleotide expression cassette according to any one of claims 9-12; preferably, the capsid protein is a wild-type or modified capsid protein selected from any one of the capsid proteins of AAV1, AAV2, AAV3, AAV4, AAV5, AAV6, AAV7, AAV8, AAV9, AAV10, AAV11, and AAV12.

16. The recombinant adeno-associated virus according to claim 15, comprising:
(i) an rAAV capsid protein, wherein the capsid protein is an AAV8 capsid protein or a modified AAV8 capsid protein; and
(ii) the polynucleotide expression cassette according to any one of claims 9-12.

17. The recombinant adeno-associated virus according to claim 16, wherein the capsid protein is a modified AAV8 capsid protein, and the modified AAV8 capsid protein comprises a substitution at amino acid positions 588-592 of the parental AAV8 by a polypeptide sequence as set forth in SEQ ID NO: 11.

18. A host cell, wherein the host cell comprises the nucleic acid according to any one of claims 1-8, or the polynucleotide expression cassette according to any one of claims 9-12, or the plasmid according to claim 13 or 14, or the recombinant adeno-associated virus according to any one of claims 15-17.

19. A pharmaceutical composition, which comprises the nucleic acid according to any one of claims 1-8, or the polynucleotide expression cassette according to any one of claims 9-12, or the plasmid according to claims 13 or 14, or the recombinant adeno-associated virus according to any one of claims 15-17, and a pharmaceutically acceptable excipient; preferably, the pharmaceutical composition is prepared as a formulation for intravitreal injection, subretinal injection, suprachoroidal space injection, intravenous injection, intratumoral injection, or intramuscular injection.

20. Use of the nucleic acid according to any one of claims 1-8, or the polynucleotide expression cassette according to any one of claims 9-12, or the plasmid according to claims 13 or 14, or the recombinant adeno-associated virus according to any one of claims 15-17 in the manufacture of a medicament for treating a VEGF-associated disease; preferably, the VEGF-associated disease is an ocular neovascular disease; more preferably, the ocular neovascular disease is selected from age-related macular degeneration, diabetic retinopathy, diabetic macular edema, central retinal vein occlusion, macular edema caused by branch retinal vein occlusion, macular edema secondary to retinal vein occlusion, polypoidal choroidal vasculopathy, wet age-related macular degeneration with very low vision, and choroidal neovascularization secondary to pathological myopia.

21. A method for treating a VEGF-associated disease in a mammalian subject, wherein the method comprises delivering a therapeutically effective amount of the nucleic acid according to any one of claims 1-8, or the polynucleotide expression cassette according to any one of claims 9-12, or the plasmid according to claims 13 or 14, or the recombinant adeno-associated virus according to any one of claims 15-17; preferably, the VEGF-associated disease is an ocular neovascular disease; more preferably, the ocular neovascular disease is selected from age-related macular degeneration, diabetic retinopathy, diabetic macular edema, central retinal vein occlusion, macular edema caused by branch retinal vein occlusion, macular edema secondary to retinal vein occlusion, polypoidal choroidal vasculopathy, wet age-related macular degeneration with very low vision, and choroidal neovascularization secondary to pathological myopia.

22. The method according to claim 21, wherein the nucleic acid, the polynucleotide expression cassette, the plasmid, or the recombinant adeno-associated virus is administered via intravitreal injection, subretinal injection, suprachoroidal space injection, preferably via suprachoroidal space injection.

23. The method according to claim 22, wherein the recombinant adeno-associated virus is administered at a dose of E8/eye to E16/eye, preferably 5E11/eye to 2E12/eye, more preferably 2E12/eye.
